# EUROPEAN PATENT SPECIFICATION

(11) **EP 3 575 391 B1**
(45) Date of publication and mention of the grant of the patent: **16.02.2022**
(21) Application number: 18175536.4
(22) Date of filing: 01.06.2018
(51) Int. Cl.: C12N 5/00, A61L 27/52, C12N 5/071

(54) **HYDROGELS FOR CULTIVATING PANCREATIC ORGANOIDS**
HYDROGELE ZUR KULTIVIERUNG VON PANKREATISCHEN ORGANOIDEN
HYDROGELS POUR LA CULTURE D'ORGANOÏDES PANCRÉATIQUES

(43) Date of publication of application: 04.12.2019
(73) Proprietor: Cellendes GmbH, 72770 Reutlingen (DE)
(72) Inventor: ANGRES, Brigitte, 72127 Kusterdingen (DE); RISCHERT, Nadine, 72760 Reutlingen (DE); WURST, Helmut, 72127 Kusterdingen (DE)
(74) Representative: Schrell, Andreas

(56) References cited:
- WO-A1-2017/036533
- WO-A2-2012/009682
- Anonymous: "3-D Life ToGro Hydrogel", , 1 January 2016 (2016-01-01), XP055515264, Retrieved from the Internet: URL:http://cellendes.com/pdfs/pds3/pds_G94 -1.pdf [retrieved on 2018-10-15]
- Angres Brigitte ET AL: "3-D Life biomimetic hydrogels: A modular system for cell environment design", , 3 March 2017 (2017-03-03), XP055515278, Retrieved from the Internet: URL:https://onlinelibrary.wiley.com/doi/pd f/10.1002/9781118851647.ch9 [retrieved on 2018-10-15]
- Anonymous: "Periodic Reporting for period 1 - LSFM4LIFE (Production and characterization of endocrine cells derived from human pancreas organoids for the cell-based therapy of type 1 diabetes) | Report Summary | LSFM4LIFE | H2020 | CORDIS | European Commission", , 2 March 2018 (2018-03-02), XP055515272, Retrieved from the Internet: URL:https://cordis.europa.eu/result/rcn/21 4741_en.html [retrieved on 2018-10-15]
- Chiara Greggio: "Artificial three-dimensional niches deconstruct pancreas development in vitro", , 1 November 2013 (2013-11-01), XP055434933, DOI: 10.1242/dev.096628 Retrieved from the Internet: URL:https://www.ncbi.nlm.nih.gov/pmc/artic les/PMC4007719/?report=printable [retrieved on 2017-12-13]

## Description

The present invention relates to the technical field of tissue engineering and cell-based therapeutic agents originating from in vitro cultivated isolated cells. The invention provides an in vitro 3D-culture system based on a chemically defined hydrogel scaffold as artificial extracellular matrix for the cultivation and expansion of pancreas-type 3D-organoids from isolated pancreatic cells.

The pancreas is a glandular organ in the digestive and endocrine system of vertebrates. It is producing several important hormones, including insulin, glucagon, for blood sugar control, as well as somatostatin and pancreatic polypeptide. The pancreas is also a digestive organ, secreting pancreatic juice and digestive enzymes that neutralize acidity and assist digestion and absorption of nutrients in the small intestine. The pancreas thus contains both, endocrine and exocrine tissue: Endocrine cells are present in the pancreatic islets (islets of Langerhans), exocrine cells with secretory function are present in the acini. All endocrine, acinar and ductal cells arise from common precursors in epithelial structure. Adult ductal cells share some similarities with embryonic primitive ducts and may retain the ability to generate endocrine cells in the adult. Adult ductal cells have been proposed to be pancreatic stem cells. Differentiation of cells into pancreatic cells proceeds through distinct pathways, corresponding to the dual endocrine and exocrine functions of the pancreas. For example, progenitor cells of the exocrine pancreas develop to exocrine acini, multi-potent pancreatic progenitor cells have the capacity to differentiate to any of the pancreatic cells, such as acinar cells, endocrine cells, and ductal cells. Pancreatic progenitor cells are basically characterized by the co-expression of the transcription factors PDX1 and NKX6-1.

Provided that the individual takes insulin for proper regulation of blood glucose concentration and pancreatic enzymes supplements to aid digestion, it is possible for an animal or human individual to live without a pancreas. Nevertheless, approaches of tissue engineering are underway to provide engineered pancreatic tissue to replace lost pancreas function in an animal or human patient, in particular by means of implanting engineered pancreatic tissue as a therapeutic agent into the patient's body. However, such cellular therapy for diabetes is currently limited by the supply of pancreatic beta cells. The production of such beta cells in vitro requires that isolated pancreatic cells can be expanded into great numbers of cells, and that the thus cultivated beta cells maintain their glucose-responsive, insulin-producing capability after re-insertion into the patient.

In tissue engineering, organs or organ-like structures can be produced in vitro in the form of so called organoids, a miniaturized and simplified version of an organ produced in vitro, derived from one or a few cells particularly from adult tissue, from embryonic stem cells, or from induced pluripotent stem cells, which then selforganize into a three-dimensional (3D) culture: a basic layered structure of interacting multiple tissue types and organ-like function. Organoids can thus generally be defined as a collection of organ-specific cell types, that have developed from stem cells or organ progenitor cells or even from adult cells, which selforganize through cell searching and spatially restricted linage commonly in a manner similar to in vivo and thus exhibit the properties of multiple organ-specific cell types, i.e. recapitulating one or more specific functions of the in vivo organ.

Organoid formation generally requires culturing the isolated cells in a so called 3D-medium which shall resemble the extra-cellular matrix (ECM) found in vivo. For example, commercial ECM products such as "Matrigel" "BME" or BME2" are a heterogeneous complex mixtures of ECM proteins, proteoglycans and growth factors secreted from mouse sarcoma cells. However, such ECM mimicking gels have compositional and structural variability and a tumour-derived matrix cannot be used in a standard clinical therapy. Thus, there is a great need for a synthetic alternative of a 3D-scaffold to replace and mimic ECM for the cultivation of organoids from isolated cells in vitro.

In an advanced therapeutic approach, the function of the pancreas is replaced or substituted by organoids that specifically resemble the pancreas organ. Such pancreatic organoids shall be provided by means of tissue engineering isolated pancreatic cells or progenitor cells thereof. However, at present methods of tissue engineering and methods for cultivating and expanding of organoids yet have failed to provide pancreatic organoids from isolated pancreatic cells, in particular from cells isolated out of adult human pancreatic tissue, in vitro that are apt for use as therapeutic agent in a patient.

One focus of interest currently lies on the cultivation and expansion to stable functional cells of isolated autologous tissue, taken from the donor, i.e. the patient. Another focus lies on the provision of engineered allogenic pancreatic tissue, in particular in conditions where patient's own pancreatic cells tend to develop cancer or in the case of autoimmune diseases. In any case, the cultivation and reliable expansion of isolated cells from adult pancreas tissue, in particular from adult human pancreas tissue for pharmaceutical application, is currently limited to particular cell culture gels of chemically undefined composition, such as Matrigel^{®} (Corning Life Sciences; BD Biosciences), Cultrex^{®}BME and Cultrex^{®}BME2 (Trevigen; AMS Biotechnology). The utilization of chemically defined biomimetic Systems remains an unsolved task.

Chemically defined hydrogel systems for three-dimensional biomimetic cell cultures are commonly based on a two-component system, consisting of a functionalized polymer as the first component and a cross-linker as the second component, which is functionalized to bind to the reactive groups of at least two polymer molecules of the first component (cross-linking), thus forming a three-dimensional scaffold of polymer molecules cross-linked with the linker molecules. The polymers are commonly functionalized with thiol (SH) reactive groups, i.e. functional groups that bind to thiol (SH) groups, for example, vinylsulfone or maleimide groups. The cross-linker molecules are functionalized to contain at least two thiol (SH) groups. Since SH-groups form stable thioether bonds with SH-reactive groups, by Michael addition, a cross-linker module containing at least two SH-groups reacts with SH-reactive groups located on two or more polymer molecules to result in large polymercross-linker networks. To obtain a neutral and biocompatible hydrogel, the two basic components, polymer molecule and cross-linker molecule are commonly present in a proportion such that the reactive groups are kept at equimolar ratios.

Although such artificial and thus chemically defined hydrogels would be well suited for use as components in therapeutic agents for animal or human patients, current cultivation techniques based on such hydrogels mostly failed in the cultivation and robust expansion of primary pancreatic cells to functional pancreatic organoids apt for use as a therapeutic agent.

In the LSFM4LIFE Report Summary "Production and characterization of endocrine cells derived from human pancreas organoids for the cell-based therapy of type 1 diabetes" (2018), it is described that biomimetic synthetic hydrogels support the growth of pancreas organoids. Angres et al. "3-D Life biomimetic hydrogels: A modular system for cell environment design" (2017) discloses biomimetic hydrogels of synthetically produced polymers having a shear modulus which can be adjusted within a range of 20-5000 Pa. 3-D hydrogels with a shear modulus of 50-200 Pa suitable for generating organoids are further known from WO 2017/036533.

The technical problem underlying the present invention is the provision of means and methods for the provision of useful therapeutics and in particular the provision of means and methods for the preparation of pancreatic 3D-organoids in vitro. The supply of adult pancreas cells expanded from isolated adult animal and in particular human tissue into pancreatic 3D-organoids in vitro gives way to efficient cell replacement therapies for the treatment of conditions and diseases of a human or animal body derived from or due to loss of function of pancreas, for example, diabetes I or diabetes II.

The problem is solved, in a first aspect, by the provision of a new composition for a hydrogel as defined in claim 1, in particular an artificial hydrogel composition for use in the cultivation and expansion of pancreatic cells isolated from adult pancreatic tissue, in particular primary endocrine progenitor cells, isolated from, in particular adult, pancreatic tissue, more specifically the pancreatic duct, into structured and functional pancreatic organoids in vitro, the composition comprising: a hydrogel with a shear modulus (G') of less than 500 Pa determined by standard rheometry at room temperature at a frequency of 1 Hz and at an angular deflection of 1% at completion of gel formation, and at least one low-molecular peptide having at least one cell adhesion motif selected from: YIGSR (SEQ ID NO: 2), GFOGER (SEQ ID NO: 3), IKVAV (SEQ ID NO: 4), wherein the peptide being linked or linkable to said hydrogel, and wherein the hydrogel composition is free of natural extracellular matrix (ECM) proteins derived from animal or human tissue, in particular free of natural adhesion proteins present in ECM of animal or human tissue.

The hydrogel of the invention is particularly characterized in a reduced cross-linking strength present in the pre-gel solution, which allows for a reduction in shear modulus (G') of the final hydrogel to 500 Pa or less. Even softer hydrogels with a shear modulus of less than 300 Pa, or less than 200 Pa may be preferred.

In particular embodiments, the artificial hydrogel composition of the invention comprises at least, or in particular consists of, the following ECM mimicking scaffold compounds:
(1.) A-functionalized polymer molecules, that is, polymer molecules that carry a plurality of binding functions of type A;
(2.) B-functionalized linker molecules, that is, linker molecules that carry a plurality of binding functions of type B, which forms bonds with the function of type A, for cross-linking a plurality of said A-functionalized polymer molecules by a plurality of said B-functionalized linker molecules, by means of A-B-bonds, that is bonds between said A-function and said B-function, to form a molecular network, a scaffold in the form of a hydrogel, and
(3.) low-molecular peptides having a cell adhesion motif selected from: YIGSR (SEQ ID NO: 2), GFOGER (SEQ ID NO: 3), IKVAV (SEQ ID NO: 4), the peptides being linked or linkable to said functionalized polymer molecules.

According to the invention, this artificial hydrogel composition is in particular characterized in that it is free of natural extracellular matrix proteins derived from animal or human tissue. In preferred embodiments the composition is further characterized in that it is free of natural adhesion proteins or proteoglycans, such as laminin-1 or other isoforms, derived from or naturally present in ECM of animal or human tissue. In particular embodiments thereof, the hydrogel composition is free of all the aforementioned natural ECM components naturally present in ECM of animal or human tissue. More particular, the hydrogel lacks presence of other ECM proteins, proteoglycans, that are present in "Matrigel"-type ECM components as secreted by Engelbreth-Holm-Swarm mouse sarcoma cells. In a preferred embodiment, the hydrogel composition of the present invention lacks any tumour-derived compound.

In the context of the present invention, the term "hydrogel" not only refers to the jellified or cured ready-to-use hydrogel product comprising crosslinked polymer and linker molecules, but also to a "pre-gel" comprising the single components, i.e. polymer and linker molecules to be brought together or already brought together in the respective proportions to generate said hydrogel by effect of the crosslinking reactions. Once polymer and linker molecules are mixed together the pre-gel in the respective proportions the hydrogel will be formed as an inevitable consequence. Thus, the respective mixture of polymer and linker molecules, the pre-gel, and hydrogel are all equivalent.

It has been surprisingly found by the inventors that a hydrogel of that particular composition is capable of not only cultivating pancreatic cells, i.e. pancreatic endocrine progenitor cells isolated from adult pancreas tissue, in particular of human origin, and to form pancreatic 3D organoids within the hydrogel scaffold, but also allows for expansion of these pancreatic organoids over several passages of cultivation to yield high counts of stable and functional pancreas organoids in vitro, without the need of other natural ECM proteins, growth factors, adhesion factors, present in other scaffold materials, for example originating from cultivated tumor cells. The present invention thus provides hydrogel compositions for use with the cultivation and expansion of pancreatic organoids, which are chemically defined and thus avoids other compounds present in ECM-substitutes known today. This allows for the preparation of cell-based therapeutics, in particular therapeutics comprising functional pancreas tissue or organoids produced de novo, for use in the clinical therapy of animal or human patients in need thereof.

Within the scope of the present invention, also other pancreatic cell types maybe cultured and expanded in the hydrogel composition and applied in the method and use of the invention as described below to form functional pancreas organoids, such further "pancreatic cells" include progenitor cells of endocrine pancreas, but also induced pluripotent stem cells (iPS) and embryonic progenitor cells or stem cells of animal origin.

In particular embodiments the A-type function is selected from vinyl sulfone (VS) and maleimide (MAL), and the B-function is thiol (SH). In a preferred variant, the A-type function is vinyl sulfone (VS). In particular embodiments, the A-functionalized polymer molecule is selected from A-functionalized polyvinyl alcohol (PVA) and A-functionalized dextran and mixtures thereof. In preferred variants, the functionalized polymer molecule is functionalized polyvinyl alcohol (PVA). In other preferred variants, the functionalized polymer molecule is a mixture of functionalized polyvinyl alcohol (PVA) and dextran. For the mixtures of functionalized polymers it is preferred that they are present in the mixture in equimolar ratio with respect to their A-type functions.

In particular embodiments, the B-functionalized linker molecule is selected from B-functionalized hyaluronic acid (HA) and B-functionalized linear molecules, comprising or consisting of polyethylene glycol (PEG). In preferred variants, the functionalized linker molecule is functionalized hyaluronic acid (HA). In other preferred variants, the functionalized linker molecule is functionalized linear polyethylene glycol (PEG).

Accordingly, in accordance with the invention a two-component hydrogel-forming composition comprises VS-PVA as the polymer and SH-HA as the linker to yield a PVA-HA hydrogel. Another such composition comprises VS-Dextran as the polymer and SH-HA as the linker to yield a dextran-HA hydrogel. Another such composition comprises VS-PVA and VS-dextran as the polymer and SH-HA as the linker to yield a PVA/detran-HA hydrogel. Another such composition comprises MAL-PVA as the polymer and SH-HA as the linker to yield a PVA-HA hydrogel. Another such composition comprises MAL-Dextran as the polymer and SH-HA as the linker to yield a dextran-HA hydrogel. Another such composition comprises MAL-PVA and MAL-dextran as the polymer and SH-HA as the linker to yield a PVA/detran-HA hydrogel. Another such composition comprises VS-PVA as the polymer and SH-PEG as the linker to yield a PVA-PEG hydrogel.

In accordance with the invention a two-component hydrogel-forming composition comprises VS-Dextran as the polymer and SH-PEG as the linker to yield a dextran-PEG hydrogel. Another such composition comprises VS-PVA and VS-dextran as the polymer and SH-PEG as the linker to yield a PVA/dextran-PEG hydrogel. Another such composition comprises MAL-Dextran as the polymer and SH-PEG as the linker to yield a dextran-PEG hydrogel. Another such composition comprises MAL-PVA as the polymer and SH-PEG as the linker to yield a PVA-PEG hydrogel. Another such composition comprises MAL-PVA and MAL-dextran as the polymer and SH-PEG as the linker to yield a PVA/dextran-PEG hydrogel.

In all variants of the embodiments of the invention the functionalized linear polyethylene glycol (PEG) is optionally covalently bound to at least one cleavable peptide linker to form a linear molecule of PEG and cleavable peptide, so called cleavable peptide linker (CD-PEG). The cleavable peptide may comprise a matrix metalloproteinase (MMOL/LOL/LP) cleavable motif. The cleavable peptide may comprise a site cleavable by trypsine. Such cleavable linker may support the separation of the cultured organoids off from the hydrogel scaffold for passage to a new charge of hydrogel for further cultivation and thus expansion on the organoids.

The A-functionalized polymer preferably has at least 3 A-functions per molecule. The A-functionalized PVA preferably has a molecular weight of more than 3000 Da. To ensure the degradability of the A-functionalized Dextran with dextranase the A-functionalized Dextran preferably has a degree of substitution of 10% or less of its glucose subunits. The A-functionalized Dextran preferably has a molecular weight of more than 10000 Da.

The B-functionalized linker molecule preferably has less than 5 functional groups per molecule. The B-functionalized linker molecule preferably has a molecular weight of more than 3000 Da.

Without wishing to be bound to the theory, these positive results of cultivation and cell proliferation and the formation of stable 3D organoids of pancreatic tissue at high counts is related to the particular composition of the hydrogel of the present invention and in particular to the concentration of the links formed between the polymer molecules and the linker molecules.

In particular embodiments of the artificial hydrogel composition the functionalized polymer molecules are cross-linked by the functionalized linker molecules at a crosslinking strength of 3 mmol/L or of less than 3 mmol/L of each of the functional groups with respect to the volume of the pre-gel solution of the hydrogel. It is preferred that the cross-linking strength is 2 mmol/L or less, more particular less than 1.8 mmol/L or 1.5 mmol/L or less than 1.5 mmol/L of the volume of the pre-gel solution of the hydrogel. Preferably, the cross-linking strength ranges from 0.3 to less than 1.8 mmol/L or from 0.5 to 1.5 mmol/L of the pre-gel solution volume. In particular embodiments, where the linker molecules consist of or comprise hyaluronic acid (HA) from as low as 0.5 mmol/L or from as low as 0.7 mmol/L, and in particular up to less than 1.8 mmol/L of the pre-gel solution volume. In particular embodiments, where the linker molecules consist of or comprise polyethylene glycol (PEG) the cross-linking strength may range from as low as 1.0 mmol/L or from as low as 1.4 mmol/L, and in particular up to less than 1.8 mmol/L of the pre-gel solution volume. In particular embodiments, the cross-linking strength may range from as low as 1.0 mmol/L or from 1.4 mmol/L up to 3 mmol/L of the pre-gel solution volume

One basic effect of the reduced cross-linking strength present in the hydrogel of the present invention is the reduction in shear modulus (G') over known hydrogels. The invention is based on hydrogels with a shear modulus (G'), as measured by standard rheology, of less than 500 Pa; a shear modulus of less than 300 Pa, or even less than 200 Pa is preferred.

In alternative embodiments, for particular applications the artificial hydrogel composition has an initial cross-linking strength of more than 2.5 mmol/L with respect to the volume of the pre-gel solution of the hydrogel. It is preferred that the initial cross-linking strength is more than 3.0 mmol/L or more than 3.5 mmol/L of the pre-gel solution volume. Preferably, the cross-linking strength ranges from 2.5 to 4.0 mmol/L or from 2.8 to 3.8 mmol/L of the pre-gel solution volume. One basic effect of the increased cross-linking strength present in the hydrogel of the present invention is the increase in shear modulus (G') over known hydrogels. An initial shear modulus (G') of more than 1000 Pa or more than 1500 Pa is preferred, but may be reduced to lower values, i.e. softer gels, due to cleavage or "digestion" of the hydrogel polymer molecules, such as dextran, and/or cleavage of the linker molecules, such as CD-PEG linker.

It has been further found that the beneficial effect of the hydrogel of the present invention on cultivation and expansion can be further supported and even enhanced by the provision of a functionalized low molecular cell adhesion peptide mimicking binding motifs of the natural extra cellular matrix (ECM), by comprising or consisting of a short binding motif. In particular embodiments, the low-molecular adhesion peptide is present in the hydrogel composition in a concentration of more than 0.5 mmol/L with respect to the volume of the pre-gel solution of the hydrogel. In particular embodiments, the functionalized adhesion peptide is present at a concentration of more than 0.8 mmol/L, of more than 1.0 mmol/L or of more than 1.5 mmol/L of the pre-gel solution volume of the hydrogel. In particular embodiments the functionalized adhesion peptide is present at a concentration of more than 0.5 mmol/L to 1.5 mmol/L at maximum.

In particular embodiments, the low molecular cell adhesion peptide is functionalized to bind to the corresponding function on the polymer component of the hydrogel composition, thus has a B-type function to bind to the A-type function of the polymer molecule. The B-type function preferably is thiol (SH), which can be provided by the addition of one SH-containing amino acid to the amino acid sequence that represents the respective binding motif, as follows. The SH-amino acid preferably is cysteine (C).

Optionally, one or more spacing structures (amino acids) are added between the SH-amino acid and the binding motif to improve cell adhesion.

In such a three component system, the stoichiometry requires that the sums of the concentration of B-type functions present on the linker molecules, and the concentration of the B-type functions present on the low molecular cell adhesion peptides equals the concentration of A-type functions present at the polymer molecules. In other instances the low molecular cell adhesion peptide may be linked to the linker molecule or to the polymer molecule by other bonds, so other concentrations of components are needed. In any case, the stoichiometry of A-type and B-type functions should be 1:1.

The hydrogel composition relies on short peptide motifs mimicking a binding structure of natural ECM. According to the present invention, the cell adhesion peptide comprises or consists of at least one peptide sequence, selected from:
YIGSR (SEQ ID NO: 2), GFOGER (SEQ ID NO: 3), IKVAV (SEQ ID NO: 4).

In preferred embodiments of the present invention, the cell adhesion peptide is limited to SEQ ID NO. 2. In other preferred embodiments the cell adhesion peptide is limited to SEQ ID NO. 3. In other preferred embodiments the cell adhesion peptide is limited to SEQ ID NO. 4 According to the present disclosure, the cell adhesion peptide can also be limited to SEQ ID NO. 5.

Particular embodiments of the present disclosure include: PVA-PEG-RGD hydrogel with RGD-containing peptide as cell adhesion peptide, PVA-HA-RGD hydrogel with RGD-containing peptide as cell adhesion peptide, dextran-PEG-RGD hydrogel with RGD-containing peptide as cell adhesion peptide, and dextran-HA-RGD hydrogel with RGD-containing peptide as cell adhesion peptide. Particular embodiments of the invention include: PVA-PEG-YIGSR hydrogel with YIGSR-containing peptide as cell adhesion peptide, PVA-HA-YIGSR hydrogel with YIGSR-containing peptide as cell adhesion peptide, dextran-PEG-YIGSR hydrogel with YIGSR-containing peptide as cell adhesion peptide, and dextran-HA-YIGSR hydrogel with YIGSR-containing peptide as cell adhesion peptide. Further particular embodiments of the invention include: PVA-PEG-GFOGER hydrogel with GFOGER-containing peptide as cell adhesion peptide, PVA-HA-GFOGER hydrogel with GFOGER-containing peptide as cell adhesion peptide, dextran-PEG-GFOGER hydrogel with GFOGER-containing peptide as cell adhesion peptide, and dextran-HA-GFOGER hydrogel with GFOGER-containing peptide as cell adhesion peptide. Further particular embodiments of the invention include: PVA-PEG-IKVAV hydrogel with IKVAV-containing peptide as cell adhesion peptide, PVA-HA-IKVAV hydrogel with IKVAV-containing peptide as cell adhesion peptide, dextran-PEG-IKVAV hydrogel with IKVAV-containing peptide as cell adhesion peptide, and dextran-HA-IKVAV hydrogel with IKVAV-containing peptide as cell adhesion peptide. Further particular embodiments of the present disclosure include: PVA-PEG-GEFYFDLRLKGK hydrogel with GEFYFDLRLKGK-containing peptide as cell adhesion peptide, PVA-HA-GEFYFDLRLKGK hydrogel with GEFYFDLRLKGK-containing peptide as cell adhesion peptide, dextran-PEG-GEFYFDLRLKGK hydrogel with GEFYFDLRLKGK-containing peptide as cell adhesion peptide, and dextran-HA-GEFYFDLRLKGK hydrogel with GEFYFDLRLKGK-containing peptide as cell adhesion peptide.

In a second aspect, the hydrogel composition of the present invention is useable or used in a method for the cultivation and expansion of isolated pancreatic cells, in particular isolated pancreatic cells into pancreatic 3D organoids in vitro; i.e. the use of the artificial hydrogel composition for the cultivation and expansion of pancreatic cells isolated from adult pancreatic tissue into pancreatic organoids in vitro.

The isolated pancreatic cells are primary cells, in particular primary pancreatic progenitor cells isolated from adult pancreas tissue. In particular embodiments, the isolated pancreatic cells are progenitor cells of endocrine cells isolated from pancreatic duct tissue. In particular embodiments, the isolated pancreatic cells are of human origin, except for human embryonic cells or tissue.

The method of use comprises, or even consists exclusively of, the following steps:
- preparing or providing one or more charges of the hydrogel composition of the present invention;
- embedding isolated pancreatic cells into a first charge of said hydrogel, the isolated pancreatic cells being pancreatic endocrine progenitor cells isolated from adult pancreatic duct tissue,
- culturing the embedded pancreatic cells under conditions to form a culture of first generation of pancreatic organoids in said hydrogel,
- expanding the culture of pancreatic organoids by repeated culturing and passaging of each culture of a further generation of pancreatic organoids, each in further charges of said hydrogel, and
- obtaining an expanded culture of organoids of pancreatic tissue.

In a third aspect, the present invention provides pancreatic 3D organoids, derived from isolated primary pancreatic cells and the hydrogel composition of the present invention, in which the pancreatic 3D organoids are embedded or cultivated.

In preferred embodiments the pancreatic organoids form a cyst-like structure comprising an epithelial-like cell layer enclosing a cyst lumen. The the cells are polarized and show their apical domain towards said cyst lumen. In particular, these cells express the cell adhesion molecule E-cadherin.

In a further aspect, the present disclosure provides a therapeutic, cell-based, agent, comprising the pancreatic organoids of the invention, in particular in a pharmaceutically acceptable carrier. The therapeutic agent is free of natural extracellular matrix proteins from animal or human tissue. The therapeutic agent is for use in the treatment of a disorder or disease in a human or animal, the disorder or disease being characterized in a condition of lack or malfunction of endocrine function of pancreas organ.

In a related aspect of the present disclosure, the therapeutic cell-based agent of the invention is used in a method for treating a disorder or disease in a human or animal, wherein the disorder or disease is characterized in a condition of lack or malfunction of the endocrine function of the pancreas organ. In particular the method of treatments concerns the replacement of pancreas function in a human or animal body, in particular the treatment of diabetes I, the treatment of diabetes II and/or the treatment of a condition after removal of pancreas from the human or animal body.

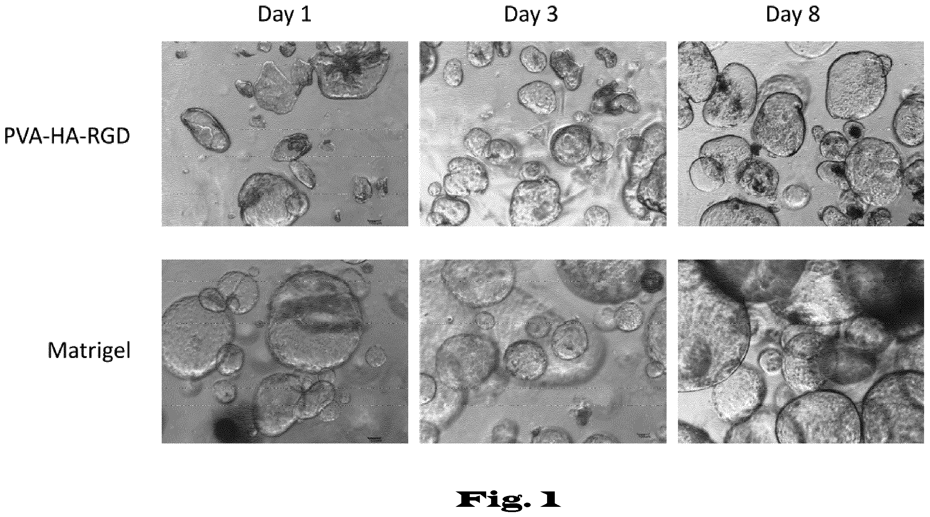

Aspects of the disclosure are further described by the present examples and figures. Figure 1 shows a comparison of mouse organoid cultures in soft PVA-HA-RGD hydrogels and Matrigel^{®} (not according to the invention). mPOs were cultured up to 8 days in soft PVA-HA-RGD hydrogels and Matrigel^{®}. One day after seeding reorganization of organoid fragments in PVA-HA-RGD gels is almost as complete as in Matrigel^{®}. After 8 days number and size of organoids in PVA-HA-RGD lag only little behind those grown in Matrigel^{®}. (Scale bar: 100 µm)

Figure 2 shows mPO expansion in PVA-PEG-RGD hydrogels (not according to the invention). mPOs were cultured in a very soft (1.8 mmol/Lol/Lol/L) PVA-PEG-RGD hydrogel. The picture (phase contrast microscopy) was taken at day 7 of passage 10. (Scale bar: 100 µm)

Figure 3 shows a comparison of human pancreatic organoid cultures in soft PVA-HA-RGD (not according to the invention). hPOs were cultured 2 days in soft PVA-HA-RGD hydrogels modified with 0.5, 1 and 2 mmol/Lol/Lol/L RGD Peptide. (Scale bar: 100 µm)

### Example 1 (not according to the present invention): Mouse pancreatic organoid culture

### 1. Mouse pancreatic growth medium

Advanced DMEM/F12 (10 mmol/L HEPES), supplemented with B-27 Supplement (1x), N-Acetylcysteine, R-spondin1 conditioned medium, Nicotinamide, mouse EGF, recombinant FGF10, Noggin, Gastrin (modified after: Boj et al. "Organoid Models of Human and Mouse Ductal Pancreatic Cancer" Cell. 2015; 160 (0): 324-338. doi: 10.1016/j.cell.2014.12.021)

### 1. Seeding mouse pancreatic organoids, previously grown in Matrigel, into PVA/SH-HA/RGD hydrogels

### 1.1 Reagents:

Mouse pancreatic growth medium: Advanced DMEM/F12 (10 mmol/L HEPES), supplemented with B-27 Supplement (1x), N-Acetylcysteine, R-spondin1 conditioned medium, Nicotinamide, mouse EGF, recombinant FGF10, Noggin, Gastrin (modified after: Boj et al. "Organoid Models of Human and Mouse Ductal Pancreatic Cancer" Cell. 2015; 160 (0): 324-338. doi: 10.1016/j.cell.2014.12.021)

SG-PVA (Cat. No. M81-3), RGD Peptide (Cat. No. 09-P-001), 10xCB pH 7,2 (Cat. No. B20-3) all obtained from Cellendes GmbH

SH-modified hyaluronic acid: hyaluronic acid was modified with SH-groups according to Burdick and Prestwich, 2011. Adv. Mater.23(12), H41-H56.

### 1.2 Gel composition:

Vinylsulfon modified PVA (VS-PVA) and SH-modified hyaluronic acid (SH-HA) crosslinked at 1 mmol/Lol/Lol/L crosslinking groups (soft hydrogel), 0.5 mmol/Lol/Lol/L RGD Peptide covalently bound to VS-PVA.

### 1.3 Protocol:

1. Mix 10xCB pH 7,2, Water, SG-PVA und RGD Peptide according to Table 1 for 2.5 gels.
2. Incubate at least 20 min at room temperature
3. Disrupt the Matrigel containing the organoids (drops in centre of the well) by scraping and pipetting up and down using a 1000 µL pipette. Transfer the organoid suspension to a 15 mL centrifuge tube and add cold Basal medium up to 10 mL. Pipette organoids up and down two times.
4. Centrifuge the tube at 100 g-200 g for 5 min at 8°C. Aspirate the supernatant, leaving ~1.5 mL of it in the tube. Resuspend the organoids in this remaining medium.
5. Use a 21-gauge needle and syringe and pull the organoid suspension up and down until organoids are completely disrupted into fragments.
6. Add cold Basal Medium to 10 ml and centrifuge the tube at 200-250 g for 5 min at 8°C.
7. Aspirate the supernatant until about 70 µL of organoid suspension is left in the tube.
8. Resuspend the pellet with a 100 µl pipette and transfer 50 µL to the gel reagent mix.
9. Add SH-HA and mix.
10. Let sit the mixture for 4 min.
11. Resuspend the mix and seed 50 µL pre-gel solution as a droplet into the centre of each required well of a 24 well plate.
12. Incubate 10 min at 37 °C.
13. Add 700 µL growth medium to each well.
14. Incubate gels at 37°C, 5% CO2.
15 ff. Change medium every two to three days.

**Table 1: Composition of hydrogel for mouse pancreatic organoid culture**

| *Gel reagents* | | | *Number of gels* | |
|---|---|---|---|---|
| | *Stock concentration* | *Final concentration* | *1.0* | *2.5* |
| | *(reactive groups)* | *(reactive groups)* | *µl* | *µl* |
| 10x CB (pH 7.2) | | | 3.00 | 7.5 |
| Water | | | 14.65 | 36,63 |
| SG-PVA | 30 mmol/L | 1.5 mmol/L | 2.50 | 6.25 |
| RGD Peptide mPo fragments in basal | 20 mmol/L | 0.5 mmol/L | 1.25 | 3.13 |
| medium | | | 20.20 | 50.00 |
| SH-HA | 5.8 mmol/L | 1 mmol/L | 8.60 | 21.50 |
| **Total** | | | **50.00** | **125.00** |

### 2. Results

### 2.1 Growth of mouse pancreatic organoids in PVA-HA-RGD hydrogels

Mouse organoids were embedded in soft hydrogels of a PVA-HA-RGD composition. One day after embedding, organoid formation proceeded more quickly than in PVA-PEG-RGD hydrogels and has almost reached the performance seen in Matrigel^{®} (Fig. 1). 8 days after seeding, organoids have reached almost the size and number as those grown in Matrigel^{®} (Fig. 1). Compared to PVA-PEG-RGD hydrogels cell death seems to be greatly reduced in this type of hydrogel and the culture easily reaches day 8 without showing many dark cell clusters. Figure 1 shows a comparison of mouse organoid cultures in soft PVA-HA-RGD hydrogels and Matrigel^{®}. mPOs were cultured up to 8 days in soft PVA-HA-RGD hydrogels and Matrigel^{®}. One day after seeding reorganization of organoid fragments in PVA-HA-RGD gels is almost as complete as in Matrigel^{®}. After 8 days number and size of organoids in PVA-HA-RGD lag only little behind those grown in Matrigel^{®}.

### 2.2 Passaging and expansion of mPO cultures

### Mechanical disruption of hydrogels for organoid passaging

Because PVA-PEG-RGD hydrogels cannot be dissolved enzymatically, a mechanic disruption of gel and organoids for the passaging of cultures was performed. In a first step hydrogels are mechanically disrupted in their culture medium with a pipet tip. The disrupted gel pieces are drawn two to three times through a 21Gx2" needle to reduce gels to small pieces and disrupt organoids into fragments. Medium is added and the suspension is centrifuged to sediment the small gel pieces and possibly loosened organoid fragments. The pellet is resuspended in an appropriate volume of medium for seeding into new gels.

### mPO Expansion in PVA-HA-RGD gels

Applying the above described passaging method mPOs cultured in PVA-HA-RGD hydrogels were split seven times every eight to nine days. mPOs were cultured this way for 56 days. A splitting ratio of 1:2 yielded an organoid amplification of approximately 128- fold.

Since the mPOs were cultured 27 passages in Matrigel^{®} before they were transferred into PVA-HA-RGD hydrogels, the total number of passages of this culture was 35. At the last passage the organoids were still growing well but the efficiency of organoid formation decreased in the last few passages.

### mPO Expansion in PVA-PEG-RGD gels

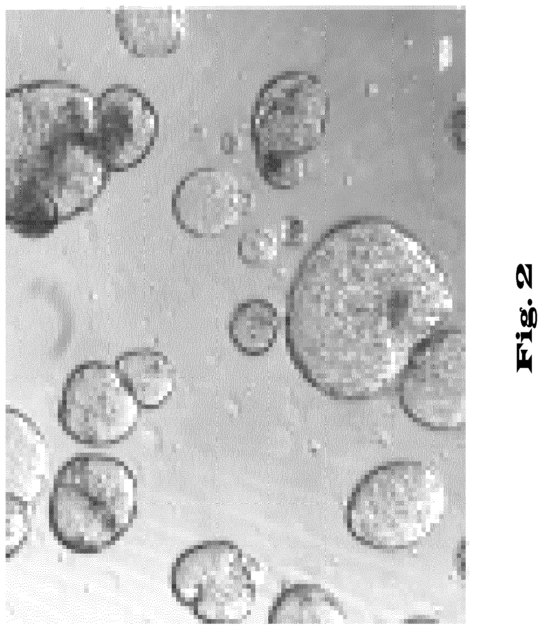

We tested whether mPOs cultured in PVA-PEG-RGD hydrogels can be mechanically passaged similary to those grown in PVA-HA-RGD hydrogels. mPOs were grown for 79 days and passaged on an average every 9 days for up to 10 passages (Fig. 2). Although not all fragments formed organoids, organoid formation was quite efficient after each split. With an average splitting ratio of 1: 4 for each gel, the total amplification of organoids was 65.000-fold after 10 passages. Figure 2 shows mPO expansion in PVA-PEG-RGD hydrogels. mPOs were cultured in a very soft (1.8 mmol/L crosslinking strength) PVA-PEG-RGD hydrogel (see Example 3). The picture (phase contrast microscopy) was taken at day 7 of passage 10.

### Cryopreservation of mPOs from biomimetic hydrogel cultures

mPOs grown in biomimetic hydrogels could be preserved using standard cryopreservation methods.

### Example 2 (not according to the present invention):Human pancreatic organoid culture

Human pancreatic organoids were grown in Cultrex BME 2, then transferred into gels with SG-PVA and SH-HA with 1 mmol/L crosslinking and a modification with 0.5 to 2 mmol/L RGD peptide.

### 1. Seeding human pancreatic organoids, previously grown in Cultrex^{®} BME, into PVA/SH-HA/RGD hydrogels

Human pancreatic growth medium: Advanced DMEM/F12 (10 mmol/L HEPES), supplemented with B-27 Supplement (1x), N-Acetylcysteine, R-spondin1 conditioned medium, Nicotinamide, mouse EGF, recombinant FGF10, Noggin, Gastrin, and others (modified after: Boj et al. "Organoid Models of Human and Mouse Ductal Pancreatic Cancer" Cell. 2015; 160 (0): 324-338. doi: 10.1016/j.cell.2014.12.021)

The procedure of seeding human pancreatic organoids grown in Cultrex BME 2 into PVA/SH-HA/RGD is the same as described for mouse pancreatic organoids. The composition of the hydrogels are given in Table 2 A-C

### 2. Results

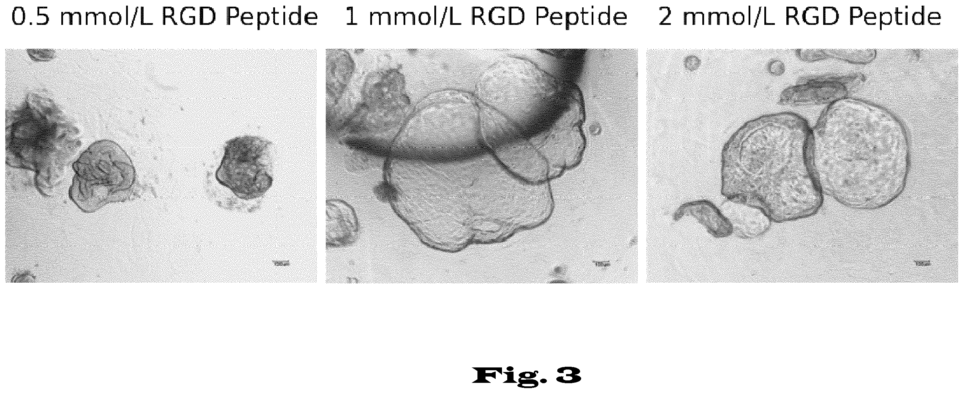

After two days of culture organoids formed in 1 mmol/L and 2 mmol/L RGD peptide-modified hydrogels. The formation of organoids was less efficient in gels with 0.5 mmol/Lol/Lol/L RGD Peptide compared to 1 mmol/L and 2 mmol/L RGD Peptide (Fig. 3).

Figure 3 shows the comparison of human pancreatic organoid cultures in soft PVA-HA-RGD. hPOs were cultured 2 days in soft PVA-HA-RGD hydrogels modified with 0.5, 1 and 2 mmol/L RGD Peptide.

Pancreas organoids consist of an epithelial-like cell layer enclosing a lumen. In this cyst-like structure cells are polarized, showing the apical domain towards the lumen and express the cell adhesion molecule E-cadherin.

**Table 2:**

| **A** | | **number of gels** | **1,00** | **2,50** |
|---|---|---|---|---|
| Gel reagents | stock concentration (reactive groups) | final concentration (reactive groups) | µl | µl |
| 10 x CB (pH 7.2) | | | 3,00 | 7,50 |
| Water | | | 14,65 | 36,63 |
| SG PVA | 30 mmol/L | 1,5 mmol/L | 2,50 | 6,25 |
| RGD Peptide | 20 mmol/L | 0,5 mmol/L | 1,25 | 3,13 |
| PO in basal medium | | | 20,00 | 50,00 |
| SH-HA | 5,8 mmol/L | 1 mmol/L | 8,60 | 21,50 |
| **Total** | | | **50,00** | **125,00** |

| **B** | | **number of gels** | **1,00** | **2,50** |
|---|---|---|---|---|
| Gel reagents | stock concentration (reactive groups) | final concentration (reactive groups) | µl | µl |
| 10 x CB (pH 7.2) | | | 3,00 | 7,50 |
| Water | | | 12,57 | 31,42 |
| SG PVA | 30 mmol/L | 2 mmol/L | 3,33 | 8,33 |
| RGD Peptide | 20 mmol/L | 1 mmol/L | 2,50 | 6,25 |
| PO in basal medium | | | 20,00 | 50,00 |
| SH-HA | 5,8 mmol/L | 1 mmol/L | 8,60 | 21,50 |
| **Total** | | | **50,00** | **125,00** |
| | | | | |

| **C** | | **number of gels** | **1,00** | **2,50** |
|---|---|---|---|---|
| Gel reagents | stock concentration (reactive groups) | final concentration (reactive groups) | µl | µl |
| 10 x CB (pH 7.2) | | | 3,00 | 7,50 |
| Water | | | 8,40 | 21,00 |
| SG PVA | 30 mmol/L | 3 mmol/L | 5,00 | 12,50 |
| RGD Peptide | 20 mmol/L | 2 mmol/L | 5,00 | 12,50 |
| PO in basal medium | | | 20,00 | 50,00 |
| SH-HA | 5,8 mmol/L | 1 mmol/L | 8,60 | 21,50 |
| **Total** | | | **50,00** | **125,00** |

### Example 3 (not according to the present invention): Shear modulus of hydrogels

### 1. Methods:

The shear moduli G' of the PVA-PEG-RGD and PVA-HA-RGD hydrogels described in Examples 1 and 2 were determined in a rheometer (Anton Paar MCR 102) at room temperature. The pregel solution was applied to the rheometer and the gel formation was followed by probing with a 25 mm plate at a frequency of 1 Hz and at an angular deflection of 1%.

### 2. Results:

After completion of gel formation, a shear modulus (G') of 10 Pa was observed for PVA-HA-RGD gels and of 250 Pa for PVA-PEG-RGD hydrogels.

## Claims

1. An artificial hydrogel composition for use in the cultivation and expansion of pancreatic cells isolated from adult pancreatic tissue into pancreatic organoids in vitro, comprising:
- a hydrogel with a shear modulus (G') of less than 500 Pa determined by standard rheometry at room temperature at a frequency of 1 Hz and at an angular deflection of 1% at completion of gel formation,
- low-molecular peptides comprising a linker function and at least one peptide sequence selected from:
YIGSR (SEQ ID NO: 2),
GFOGER (SEQ ID NO: 3),
IKVAV (SEQ ID NO: 4),
wherein the peptides are linked or linkable to said hydrogel, and wherein the hydrogel composition is free of natural extracellular matrix (ECM) proteins derived from animal or human tissue.

2. The composition of claim 1, further **characterized in that** it is free of natural adhesion proteins present in ECM of animal or human tissue.

3. The composition of claim 1 or 2, wherein said hydrogel is comprised of A-functionalized polymer molecules crosslinked by B-functionalized linker molecules at a crosslinking strength of 3 mmol/L or less than 3 mmol/L of the pre-gel solution volume of the hydrogel.

4. The composition of claim 3, wherein said A-functionalized polymer molecules are crosslinked by said B-functionalized linker molecules at a crosslinking strength of less than 1.8 mmol/L of the pre-gel solution volume of the hydrogel.

5. The composition of claims 3 or 4, wherein the A-function is selected from vinylsulfone and maleimide, and the B-function is thiol.

6. The composition of any one of claims 3 to 5, wherein the A-functionalized polymer molecule is selected from functionalized polyvinyl alcohol (PVA) and functionalized dextran.

7. The composition of any one of claims 3 to 6, wherein the B-functionalized linker molecule is selected from functionalized hyaluronic acid (HA) and functionalized linear molecules, comprising or consisting of polyethylene glycol (PEG).

8. The composition of any one of the preceding claims, wherein said functionalized adhesion peptide is present at a concentration of 0.5 mmol/L or more of hydrogel volume.

9. Use of the artificial hydrogel composition of any one of the preceding claims for the cultivation and expansion of pancreatic cells isolated from adult pancreatic tissue into pancreatic organoids in vitro.

10. The use of claim 9, wherein the isolated pancreatic cells are primary pancreatic progenitor cells isolated from adult pancreas tissue, except for human embryonic cells or tissue.

11. A method for the preparation of organoids of pancreatic tissue, comprising the steps of:
- preparing or providing one or more charges of the hydrogel composition of any one of claims 1 to 8,
- embedding isolated primary pancreatic cells into a first charge of said hydrogel, the isolated pancreatic cells being pancreatic endocrine progenitor cells isolated from adult pancreatic duct tissue,
- culturing the embedded pancreatic cells under conditions to form a culture of first generation of pancreatic organoids in said hydrogel,
- expanding the culture of pancreatic organoids by repeated culturing and passaging of each culture of a further generation of pancreatic organoids, each in further charges of said hydrogel, and
- obtaining an expanded culture of organoids of pancreatic tissue.

12. A pancreatic organoid, obtainable from isolated primary pancreatic cells from adult pancreas tissue and cultivated in the artificial hydrogel composition of any one of claims 1 to 8, wherein the pancreatic organoid is embedded in an artificial hydrogel composition of any one of claims 1 to 8.

13. The pancreatic organoid of claim 12, being a cyst-like structure comprising an epithelial-like cell layer enclosing a cyst lumen, wherein the cells are polarized and show their apical domain towards said cyst lumen.

## Patentansprüche

1. Künstliche Hydrogelzusammensetzung zur Verwendung bei der Kultivierung und Expansion von aus adultem Pankreasgewebe isolierten Pankreaszellen zu Pankreasorganoiden in vitro umfassend:
- ein Hydrogel mit einem Schermodul (G') von weniger als 500 Pa, bestimmt durch Standardrheometrie bei Raumtemperatur bei einer Frequenz von 1 Hz und bei einer Winkelablenkung von 1 % bei abgeschlossener Gelbildung,
- niedermolekulare Peptide, die eine Linkerfunktion und mindestens eine Peptidsequenz umfassen, ausgewählt aus:
YIGSR (SEQ ID NO: 2),
GFOGER (SEQ ID NO: 3),
IKVAV (SEQ ID NR: 4),
wobei die Peptide mit dem Hydrogel verbunden oder verbindbar sind, und wobei die Hydrogelzusammensetzung frei von natürlichen extrazellulären Matrixproteinen (EZM) ist, die aus tierischem oder menschlichem Gewebe stammen.

2. Die Zusammensetzung nach Anspruch 1, ferner **dadurch gekennzeichnet, dass** sie frei von natürlichen Adhäsionsproteinen ist, die in der EZM von tierischem oder menschlichem Gewebe vorhanden sind.

3. Zusammensetzung nach Anspruch 1 oder 2, wobei das Hydrogel A-funktionalisierte Polymermoleküle umfasst, die durch B-funktionalisierte Linkermoleküle mit einer Vernetzungsstärke von 3 mmol/L oder weniger als 3 mmol/L des Vorgel-Lösungsvolumens des Hydrogels vernetzt sind.

4. Zusammensetzung nach Anspruch 3, wobei die A-funktionalisierten Polymermoleküle durch die B-funktionalisierten Linkermoleküle mit einer Vernetzungsstärke von weniger als 1,8 mmol/L des Vorgel-Lösungsvolumens des Hydrogels vernetzt sind.

5. Zusammensetzung nach Anspruch 3 oder 4, wobei die A-Funktion ausgewählt ist aus Vinylsulfon und Maleimid und die B-Funktion ein Thiol ist.

6. Zusammensetzung nach einem der Ansprüche 3 bis 5, wobei das A-funktionalisierte Polymermolekül ausgewählt ist aus funktionalisiertem Polyvinylalkohol (PVA) und funktionalisiertem Dextran.

7. Zusammensetzung nach einem der Ansprüche 3 bis 6, wobei das B-funktionalisierte Linkermolekül ausgewählt ist aus funktionalisierter Hyaluronsäure (HA) und funktionalisierten linearen Molekülen, die Polyethylenglykol (PEG) umfassen oder daraus bestehen.

8. Die Zusammensetzung nach einem der vorhergehenden Ansprüche, wobei das funktionalisierte Adhäsionspeptid in einer Konzentration von 0,5 mmol/L oder mehr, bezogen auf das Hydrogelvolumen, vorhanden ist.

9. Verwendung der künstlichen Hydrogelzusammensetzung nach einem der vorhergehenden Ansprüche für die Kultivierung und Expansion von aus adultem Pankreasgewebe isolierten Pankreaszellen zu Pankreasorganoiden in vitro.

10. Verwendung nach Anspruch 9, wobei die isolierten Pankreaszellen primäre Pankreasvorläuferzellen sind, die aus adultem Pankreasgewebe isoliert wurden, ausgenommen humane embryonale Zellen oder Gewebe.

11. Verfahren zur Herstellung von Organoiden des Pankreasgewebes, das die folgenden Schritte umfasst
- Herstellung oder Bereitstellung einer oder mehrerer Chargen der Hydrogelzusammensetzung nach einem der Ansprüche 1 bis 8,
- Einbetten isolierter primärer Pankreaszellen in eine erste Charge des Hydrogels, wobei es sich bei den isolierten Pankreaszellen um endokrine Pankreasvorläuferzellen handelt, die aus dem Gewebe des adulten Pankreasgangs isoliert wurden,
- Kultivierung der eingebetteten Pankreaszellen unter Bedingungen zur Bildung einer Kultur der ersten Generation von Pankreasorganoiden in dem Hydrogel,
- Expandieren der Kultur von Pankreasorganoiden durch wiederholtes Kultivieren und Überführen jeder Kultur einer weiteren Generation von Pankreasorganoiden, jeweils in weitere Chargen des Hydrogels, und
- Erhalten einer expandierten Kultur von Organoiden des Pankreasgewebes.

12. Pankreasorganoid, erhalten aus isolierten primären Pankreaszellen aus adultem Pankreasgewebe und kultiviert in der künstlichen Hydrogelzusammensetzung nach einem der Ansprüche 1 bis 8, wobei das Pankreasorganoid in eine künstliche Hydrogelzusammensetzung nach einem der Ansprüche 1 bis 8 eingebettet ist.

13. Pankreasorganoid nach Anspruch 12, das eine zystenartige Struktur ist, die eine epithelartige Zellschicht umfasst, die ein Zystenlumen umschließt, wobei die Zellen polarisiert sind und ihren apikalen Bereich zum Zystenlumen hin zeigen.

## Revendications

1. Composition d'hydrogel artificiel pour utilisation dans la culture et l'expansion de cellules pancréatiques isolées à partir de tissu pancréatique adulte en organoïdes pancréatiques in vitro, comprenant :
- un hydrogel ayant un module de cisaillement (G') inférieur à 500 Pa déterminé par rhéométrie standard à température ambiante à une fréquence de 1 Hz et à une déviation angulaire de 1% à la fin de la formation du gel,
- peptides de faible masse moléculaire comprenant une fonction de liaison et au moins une séquence peptidique choisie parmi :
YIGSR (SEQ ID NO : 2),
GFOGER (SEQ ID NO : 3),
IKVAV (SEQ ID NO : 4),
dans laquelle les peptides sont liés ou peuvent être liés audit hydrogel, et dans laquelle la composition d'hydrogel est exempte de protéines de la matrice extracellulaire naturelle (MEC) dérivées de tissus animaux ou humains.

2. La composition de la revendication 1, **caractérisée en outre en ce qu'**elle est exempte de protéines d'adhésion naturelles présentes dans la MEC du tissu animal ou humain.

3. Composition selon la revendication 1 ou 2, dans laquelle ledit hydrogel est compris dans des molécules de polymère à fonctionnalité A réticulées par des molécules de liaison à fonctionnalité B à une force de réticulation de 3 mmol/L ou inférieure à 3 mmol/L du volume de la solution de pré-gel de l'hydrogel.

4. La composition de la revendication 3, dans laquelle lesdites molécules de polymère à fonctionnalité A sont réticulées par lesdites molécules de lieur à fonctionnalité B à une force de réticulation inférieure à 1,8 mmol/L du volume de solution prégel de l'hydrogel.

5. La composition des revendications 3 ou 4, dans laquelle la fonction A est choisie parmi la vinylsulfone et le maléimide, et la fonction B est un thiol.

6. La composition de l'une quelconque des revendications 3 à 5, dans laquelle la molécule de polymère à fonctionnalité A est choisie parmi l'alcool polyvinylique (PVA) fonctionnalisé et le dextrane fonctionnalisé.

7. La composition de l'une quelconque des revendications 3 à 6, dans laquelle la molécule de liaison à fonctionnalité B est choisie parmi l'acide hyaluronique (HA) fonctionnalisé et les molécules linéaires fonctionnalisées, comprenant ou consistant en du polyéthylène glycol (PEG).

8. La composition de l'une quelconque des revendications précédentes, dans laquelle ledit peptide d'adhésion fonctionnalisé est présent à une concentration de 0,5 mmol/L ou plus du volume de l'hydrogel.

9. Utilisation de la composition d'hydrogel artificiel de l'une quelconque des revendications précédentes pour la culture et l'expansion de cellules pancréatiques isolées à partir de tissu pancréatique adulte en organoïdes pancréatiques in vitro.

10. L'utilisation de la revendication 9, dans laquelle les cellules pancréatiques isolées sont des cellules progénitrices pancréatiques primaires isolées à partir de tissu pancréatique adulte, à l'exception des cellules ou du tissu embryonnaire humain.

11. Procédé de préparation d'organoïdes de tissu pancréatique, comprenant les étapes consistant à :
- préparer ou fournir une ou plusieurs charges de la composition d'hydrogel de l'une quelconque des revendications 1 à 8,
- encastrer des cellules pancréatiques primaires isolées dans une première charge dudit hydrogel, les cellules pancréatiques isolées étant des cellules progénitrices endocrines pancréatiques isolées à partir de tissu de conduit pancréatique adulte,
- cultiver les cellules pancréatiques encastrées dans des conditions permettant de former une culture de première génération d'organoïdes pancréatiques dans ledit hydrogel,
- l'expansion de la culture d'organoïdes pancréatiques par la culture et le passage répétés de chaque culture d'une autre génération d'organoïdes pancréatiques, chacun dans d'autres charges dudit hydrogel, et
- obtenir une culture expansée d'organoïdes de tissu pancréatique.

12. Organoïde pancréatique, pouvant être obtenu à partir de cellules pancréatiques primaires isolées provenant de tissu pancréatique adulte et cultivé dans la composition d'hydrogel artificiel de l'une quelconque des revendications 1 à 8, dans lequel l'organoïde pancréatique est encastré dans une composition d'hydrogel artificiel de l'une quelconque des revendications 1 à 8.

13. L'organoïde pancréatique de la revendication 12, étant une structure de type kyste comprenant une couche de cellules de type épithélial enfermant une lumière de kyste, dans lequel les cellules sont polarisées et montrent leur domaine apical vers ladite lumière de kyste.
